# FASCICULE DE BREVET EUROPEEN

(11) **EP 1 638 480 B1**
(45) Date de publication et mention de la délivrance du brevet: **23.07.2008**
(21) Numéro de dépôt: 04787333.6
(22) Date de dépôt: 08.09.2004
(51) Int. Cl.: A61F 2/00, D04B 21/02, D04B 21/04, D04B 21/12

(54) **TRICOT PROTHETIQUE A PROPRIETES VARIABLES**
PROTHETISCHES GEWIRKE MIT VARIABLEN EIGENSCHAFTEN
PROSTHETIC KNIT FABRIC WITH VARIABLE PROPERTIES

(30) Priorité: 16.09.2003 FR 0310853
(43) Date de publication de la demande: 29.03.2006
(73) Titulaire: SOFRADIM PRODUCTION, 01600 Trévoux (FR)
(72) Inventeur: THERIN, Michel, F-69004 Lyon (FR); MENEGHIN, Alfredo, F-69009 Lyon (FR)
(74) Mandataire: Brédeville, Odile Marie
(86) Numéro de dépôt international: PCT/FR2004/002283
(87) Numéro de publication internationale: WO 2005/027786

(56) Documents cités:
- WO-A-01/80773
- WO-A-20/04004600
- FR-A- 2 779 937

## Description

La présente invention concerne un tricot prothétique à propriétés variables, en particulier à résistance mécanique et densité variables, selon au moins une dimension ou direction d'extension dudit tricot, utilisable notamment sous forme de bandes ou bandelettes découpées dans ledit tricot dans le traitement des troubles de la statique pelvienne principalement chez la femme, également appelés prolapsus. Ce renfort peut également être utilisé pour préparer des bandelettes de soutènement urétral dans le traitement de l'incontinence d'effort chez la femme.

Le traitement chirurgical des prolapsus implique l'utilisation de renforts sous la forme d'implants pour soutenir l'organe prolabé. La partie centrale de l'implant est disposée au niveau de cet organe et peut être en contact avec ce dernier afin de le soutenir alors que les parties latérales de l'implant sont fixées à des éléments anatomiques stables tels que la paroi abdominale, la membrane obturatrice, le promontoire du sacrum, les ligaments sacro-sciatiques ou le pubis, par exemple au moyen d'agrafes, de sutures ou par simple ancrage tissulaire.

De façon connue, un tel implant de soutien doit satisfaire à de nombreuses contraintes, et en particulier présenter une résistance mécanique adaptée dans toutes les directions, être biocompatible, souple, et présenter néanmoins une certaine élasticité. Ces implants de soutien doivent de préférence être poreux et non agressifs dans leur partie centrale soutenant l'organe. Ces implants de soutien doivent être suturables. Enfin, il est souhaitable que ces implants de soutien soient adaptés à la morphologie du patient.

Des mises en forme de textiles de façon à se conformer, d'une part, à l'anatomie de la région sous-urétrale et, d'autre part, à la technique chirurgicale employée sont connues.

Les documents WO01/52750 et WO02/28312 décrivent une bandelette de soutènement sous-urétral comprenant un tricot ajouré, ce tricot étant composé de deux nappes de fils sur l'ensemble de la surface de la bandelette. Le tricot réalisé est uniforme et la bandelette présente ainsi les mêmes propriétés de porosité, de densité, d'élasticité et de résistance mécanique sur l'ensemble de sa surface, c'est-à-dire et dans sa partie centrale et au niveau de ses extrémités.

Les techniques chirurgicales de traitement des prolapsus et de l'incontinence urinaire d'effort font de plus en plus appel à des renforts présentant une zone centrale plus large, se plaçant sous l'organe à soutenir, que les extrémités latérales destinées à s'ancrer à distance dans les éléments musculo-aponévrotiques Les propriétés mécaniques et biologiques attendues de ces parties distinctes sont différentes.

Au niveau de leurs extrémités, ces implants de soutien doivent pouvoir être découpés en bandelettes étroites de l'ordre de 1 cm sans se démailler ni tuiler. Par « tuilage », on entend, au sens de la présente demande, l'enroulement spontané de la bandelette sur elle-même, autour de son axe longitudinal, sous contrainte en tension selon sa longueur. Ces implants doivent conserver sous cette forme des propriétés mécaniques, notamment en résistance, suffisantes, tout en limitant au maximum le relargage de particules, c'est-à-dire de bouts de fils, lorsqu'elles sont sous contrainte et permettre un ancrage tissulaire mécaniquement stable.

En revanche, au niveau de la partie centrale de l'implant, qui est la partie soutenant l'urètre ou l'organe prolabé, il est particulièrement important que l'implant présente une densité minimale, une porosité maximale et une grande souplesse et une grande élasticité, la résistance à la déchirure et au tuilage dans cette partie, non appelée à être découpée en fines bandelettes, étant moins importante qu'au niveau des extrémités. En effet, cette partie centrale étant susceptible de se trouver en contact avec l'organe à soutenir, elle doit être de préférence peu agressive vis-à-vis de la paroi des viscères creux tels que la vessie, le vagin, le rectum ou l'urètre afin de minimiser les risques d'érosion.

Ces implants, et en particulier les bandelettes qu'on peut y découper, doivent également de préférence être indémaillables.

Enfin, les patients présentant des morphologies différentes, il est important que le chirurgien puisse façonner l'implant sur le site opératoire, au moment de l'intervention, pour pouvoir adapter sa configuration ou forme à celle de l'organisme du patient.

Ainsi, il serait intéressant de disposer d'un tricot, découpable à volonté, présentant une zone particulièrement peu dense, à larges pores, souple et élastique, qui pourrait correspondre à la partie centrale de l'implant, et des zones particulièrement résistantes, qui pourraient correspondre aux extrémités latérales de l'implant, et pouvant être découpées dans des largeurs par exemple de moins d'1 cm de large tout en résistant au tuilage et au démaillage.

Le document WO01/80773 décrit un tricot prothétique ajouré, dont la partie centrale présente une élasticité supérieure à celle présentée par les parties périphériques, la partie centrale étant destinée à être déformée pour réaliser une protubérance. Toutefois, ce tricot est réalisé selon un arrangement de plusieurs nappes de fils, dont une seule est maillante, cette dernière étant une armure chaînette. Par « nappe maillante », on entend selon la présente demande, une nappe de fils pour laquelle le barème suivi pour le tricotage des fils conduit à la formation de mailles. Le tricot décrit dans WO01/80773, du fait qu'il ne comprend qu'une seule nappe maillante selon une armure chaînette, est déséquilibré et ne permet pas, dans ses parties périphériques, la découpe de parties indémaillables de petite largeur, telles que par exemple égale à 1 cm de large, résistantes à la déchirure et au tuilage. Le document FR 2779937 décrit un tricot prothétique ajouré fait d'une seule pièce, à base d'un arrangement constitué d'un matériau polymère biocompatible.

La présente invention vise à remédier à ce problème en proposant de manière générale un tricot à densité variable selon au moins une direction ou dimension dudit tricot, prêt à être découpé, particulièrement utile pour la réalisation ou obtention, simple et rapide, d'implants de soutien pour le traitement des prolapsus et de l'incontinence urinaire d'effort, comprenant une bande centrale, dans le sens de la fabrication du tricot, présentant faible densité, large porosité, élasticité et souplesse et deux bandes latérales, de part et d'autre de la bande centrale, présentant chacune une densité supérieure procurant une bonne résistance à la déchirure et au tuilage, ce tricot étant réalisé d'une seule pièce et ne présentant aucune surépaisseur ni discontinuité d'une bande à l'autre, et autorisant par ailleurs le découpage de bandelettes ayant des extrémités de petite largeur, comme par exemple allant de 1 cm à 3 cm.

La présente invention porte sur un tricot prothétique ajouré fait d'une seule pièce, à base d'un arrangement constitué par plusieurs nappes de fils d'un matériau polymère biocompatible, comprenant, de manière continue dans le sens de la fabrication du tricot, une bande centrale et deux bandes latérales respectivement de part et d'autre de la bande centrale, l'élasticité du tricot dans la bande centrale étant supérieure à l'élasticité du tricot dans chacune des bandes latérales, caractérisé en ce qu'il comprend i) au moins deux nappes de base, une nappe avant et une nappe arrière, s'étendant au moins sur l'ensemble de la surface de la bande centrale, lesdites deux nappes de base étant des nappes maillantes et définissant une première armure, et ii) au moins deux nappes supplémentaires, en arrière de ladite nappe de base arrière, s'étendant seulement sur les surfaces respectives des bandes latérales, lesdites deux nappes supplémentaires étant des nappes non maillantes de trame partielle et définissant une deuxième armure.

La présente invention porte également sur un procédé de fabrication d'un tricot prothétique ajouré fait d'une seule pièce, à base d'un arrangement constitué par plusieurs nappes de fils d'un matériau polymère biocompatible, comprenant, de manière continue, dans le sens de la fabrication du tricot, une bande centrale et deux bandes latérales, respectivement de part et d'autre de la bande centrale, l'élasticité du tricot dans la bande centrale étant supérieure à l'élasticité du tricot dans chacune des bandes latérales, caractérisé en ce qu'il comprend l'étape suivante :
- fabrication d'un tricot sur un métier chaîne ou Rachel selon deux nappes de base, une nappe de base avant et une nappe de base arrière définissant une première armure, et deux nappes supplémentaires définissant une deuxième armure, les deux nappes de base étant enfilées en continu ou à disposition au moins sur la largeur de la bande centrale, chacune des nappes de base étant obtenue à partir d'une barre à passettes, le barème suivi pour le tricotage des fils de chaque nappe de base conduisant à la formation de mailles, les deux nappes supplémentaires étant enfilées à disposition seulement sur les largeurs respectives des bandes latérales, chacune des nappes supplémentaires étant obtenue à partir d'une barre à passettes, le barème suivi pour le tricotage des fils de chaque nappe supplémentaire conduisant à une trame partielle non maillante.

Le tricot selon l'invention permet de découper facilement des implants de soutien présentant une partie centrale particulièrement peu dense, poreuse, souple et élastique et des extrémités particulièrement résistantes à la déchirure et au tuilage, même si ces extrémités présentent une largeur allant de 1 cm à 3 cm par exemple.

Du fait de la présence d'au moins deux nappes maillantes sur la bande centrale du tricot, ce dernier est particulièrement stable.

Dans une forme préférée de réalisation de l'invention, les deux nappes de base s'étendent sur la totalité de la surface du tricot, c'est-à-dire sur la surface de la bande centrale et sur les surfaces respectives des bandes latérales. Dans ce cas, du fait de la présence d'au moins deux nappes maillantes sur l'ensemble du tricot, ce dernier est particulièrement équilibré et stabilisé dimensionnellement sur toute sa surface, c'est-à-dire qu'il ne risque pas de perdre de ses qualités minimum d'élasticité et de résistance, dans quelque direction ou dimension que ce soit, longitudinale, transversale ou diagonale, et pour quelque bande que ce soit, centrale ou latérale, après découpage.

Par ailleurs, du fait du tricotage spécifique du tricot par ajout, sur deux nappes de base, de deux nappes à trame partielle sur les parties latérales, le tricot selon l'invention est réalisé en une seule pièce et ne présente aucune surépaisseur ou discontinuité d'aspect d'une bande à l'autre.

Dans la présente demande, par « tricot prothétique », on entend un tricot destiné à être implanté dans le corps humain ou animal sous la forme d'une prothèse ou de toute autre pièce façonnée au moins en partie avec ledit tricot.

Dans la présente demande, par « tricot ajouré », on entend un tricot dont l'armure ou les armures déterminent des alvéoles ou des vides dans l'épaisseur du tricot, ces alvéoles ou vides pouvant constituer des canaux débouchant de part et d'autre du tricot. Un tel tricot ajouré permet une meilleure intégration tissulaire. En particulier, du fait de son tricotage spécifique, le tricot prothétique selon l'invention est ajouré sur l'ensemble des ses bandes centrale et latérales. En particulier, la porosité du tricot dans la bande centrale est supérieure à la porosité du tricot dans chacune des bandes latérales.

Dans la présente demande,
- la masse surfacique d'un tricot est mesurée selon la norme ISO 3801,
- la force à la rupture d'un tricot dans la direction longitudinale et dans la direction transversale est mesurée selon la norme ISO 13934-1.

Dans une forme préférée de réalisation de l'invention, la masse surfacique, ou encore la densité, du tricot dans chacune des bandes latérales est supérieure à la masse surfacique, ou encore densité, du tricot dans la bande centrale. Dans une forme préférée de réalisation de l'invention, la masse surfacique du tricot dans chacune des bandes latérales est au moins deux fois supérieure à la masse surfacique du tricot dans la bande centrale.

Dans une forme préférée de réalisation de l'invention, la force à la rupture du tricot dans la direction longitudinale et dans la direction transversale au niveau de chaque bande latérale est au moins deux fois supérieure à la force à la rupture du tricot dans la direction longitudinale et dans la direction transversale au niveau de la bande centrale.

Ainsi, le tricot selon l'invention présente une élasticité dans sa bande centrale supérieure à celle qu'il présente dans chacune des bandes latérales.

Dans une forme de réalisation de l'invention, la bande centrale présente une masse surfacique allant de 15 à 50 g/m², et de préférence allant de 25 à 35 g/m². De préférence, la force à la rupture du tricot dans la bande centrale est d'environ 90 N dans la direction longitudinale et dans la direction transversale.

Dans une forme de réalisation de l'invention, chaque bande latérale présente une masse surfacique allant de 60 à 80 g/m², et de préférence de l'ordre de 70 g/m². De préférence, la force à la rupture du tricot dans chaque bande latérale est d'environ 200 N dans la direction longitudinale et dans la direction transversale.

De préférence, le tricot selon l'invention est réalisé à base de fils, monofilaments ou multifilaments, d'un matériau polymère biocompatible choisi parmi le polypropylène, le polyester, le polyamide et leurs mélanges.

De préférence, les fils monofilaments présentent un diamètre allant de préférence de 0,06 mm à 0,18 mm. De préférence, les fils multifilaments présentent un titre allant de 44 dtex à 100 dtex.

La première armure du tricot selon l'invention est définie par une nappe de base avant et une nappe de base arrière, chacune de ces nappes étant obtenue par exemple à partir d'une barre à passettes d'un métier chaîne ou Rachel, le barème suivi pour le tricotage des fils de chaque nappe conduisant à la formation de mailles. Dans une forme préférée de réalisation de l'invention, ce barème conduit à la formation d'un motif atlas à mailles ouvertes. Par motif « atlas », on entend, au sens de la présente demande, un motif obtenu par le fait que chaque passette jette son fil sous une ou plusieurs aiguilles, plusieurs fois dans le même sens, puis en sens inverse. De préférence encore, ce barème conduit à la formation d'un motif atlas à mailles ouvertes irrégulier. Par « atlas irrégulier », on entend, au sens de la présente demande, un motif atlas dont les mailles consécutives sont formées sur des colonnes espacées irrégulièrement.

Dans une forme préférée de réalisation de l'invention, les fils de la nappe de base avant sont tricotés selon un barème 0-1/1-2/3-4/5-4/4-3/2-1// et les fils de la nappe de base arrière sont tricotés selon un barème 5-4/4-3/2-1/0-1/1-2/3-4//. De préférence, les deux barres à passette des nappes de base sont enfilées en continu, c'est-à-dire sur la largeur totale du tricot selon la dimension transversale, un plein, un vide, autrement dit une passette pleine, une passette vide, et elles se déplacent symétriquement l'une par rapport à l'autre.

La deuxième armure du tricot selon l'invention est définie par deux nappes supplémentaires, en arrière de la nappe de base arrière, chacune de ces deux nappes supplémentaires étant obtenue par exemple à partir d'une barre à passettes d'un métier chaîne ou Rachel, le barème suivi pour le tricotage des fils de chaque dite nappe supplémentaire conduisant à une trame partielle non maillante, et par conséquent ne conduisant pas à la formation de mailles.

Dans une forme préférée de réalisation de l'invention, la première armure s'étend sur l'ensemble de la surface du tricot et les deux barres à passettes des nappes supplémentaires se déplacent en trame partielle avec des jetées « sous » en rapport avec la première armure. De préférence encore, les deux barres à passette des nappes supplémentaires se déplacent en trame partielle avec des jetées « sous » deux et cinq aiguilles. Grâce à ce déplacement en trame, il est possible d'améliorer la résistance transversale du tricot au niveau des bandes latérales tout en gardant une masse surfacique peu élevée.

Dans une forme préférée de réalisation de l'invention, les fils de l'une des deux nappes supplémentaires sont tricotés selon le barème 3-3/5-5/0-0/2-2/0-0/5-5// et les fils de l'autre nappe supplémentaire sont tricotés selon le barème 2-2/0-0/5-5/3-3/5-5/0-0//.

De préférence, les deux barres à passettes des nappes supplémentaires sont enfilées à disposition, c'est-à-dire seulement sur les largeurs respectives, selon la dimension transversale du tricot, des bandes latérales. A l'intérieur de chaque bande latérale, les deux barres à passettes des nappes supplémentaires sont de préférence enfilées un plein, un vide.

Grâce à l'ajout de deux nappes supplémentaires obtenues à partir de barres de trame partielle sur les parties latérales du tricot, l'épaisseur du tricot est sensiblement égale sur toute la surface du tricot prothétique. Cette épaisseur va de préférence de 0,3 mm à 0,5 mm.

Le tricot prothétique peut comprendre en outre d'autres nappes de fils en addition aux deux nappes de base et aux deux nappes supplémentaires déjà décrites ci-dessus. Dans ce cas, on rajoute sur le métier à tricoter des barres à passettes additionnelles pour obtenir autant de nappes de fils additionnelles. Par exemple, le tricot prothétique selon l'invention peut comprendre deux nappes de base et trois nappes supplémentaires ou encore quatre nappes supplémentaires.

Un autre objet de l'invention est l'utilisation d'un tricot prothétique tel que défini ci-dessus pour obtenir un produit prothétique à usage chirurgical, notamment pour obtenir un implant de soutien pour le traitement des prolapsus et de l'incontinence urinaire d'effort.

Un autre objet de l'invention est un implant de soutien pour le traitement des prolapsus et de l'incontinence urinaire d'effort obtenu par découpage d'un tricot prothétique tel que défini ci-dessus.

Grâce aux propriétés particulières d'une part de la bande centrale du tricot, à savoir faible masse surfacique, grande porosité, élasticité et souplesse, et d'autre part de ses bandes latérales, à savoir résistance à la rupture et au tuilage, il est possible, en particulier pour le chirurgien, de découper directement dans ce tricot un implant de soutien pour le traitement des prolapsus et de l'incontinence urinaire d'effort présentant dans sa partie centrale faible densité, porosité, élasticité et souplesse et au niveau de ses extrémités résistance à la déchirure, ces extrémités ne tuilant pas, même découpées en fines largeurs, par exemple de l'ordre d'1 cm de large.

L'invention sera mieux comprise de la description qui suit, en référence au dessin annexé:
- la figure 1 représente un tricot prothétique selon l'invention, comprenant des pré-découpes pour la réalisation d'implants de soutien pour le traitement des prolapsus et de l'incontinence urinaire d'effort de différentes formes,
- la figure 2 représente un dessin schématique simplifié d'un fil de la nappe de base avant (A) et d'un fil de la nappe de base arrière (B) d'une première armure d'un tricot selon l'invention,
- la figure 3 représente un dessin schématique simplifié de la première armure selon la figure 2,
- la figure 4 représente un dessin schématique simplifié d'un fil (C) d'une première nappe supplémentaire et d'un fil (D) d'une deuxième nappe supplémentaire, d'une deuxième armure d'un tricot selon l'invention,
- la figure 5 représente un dessin schématique simplifié de la deuxième armure selon la figure 4,
- la figure 6 représente un dessin schématique simplifié de la combinaison de la première armure et de la deuxième armure selon respectivement les figures 2 et 4.

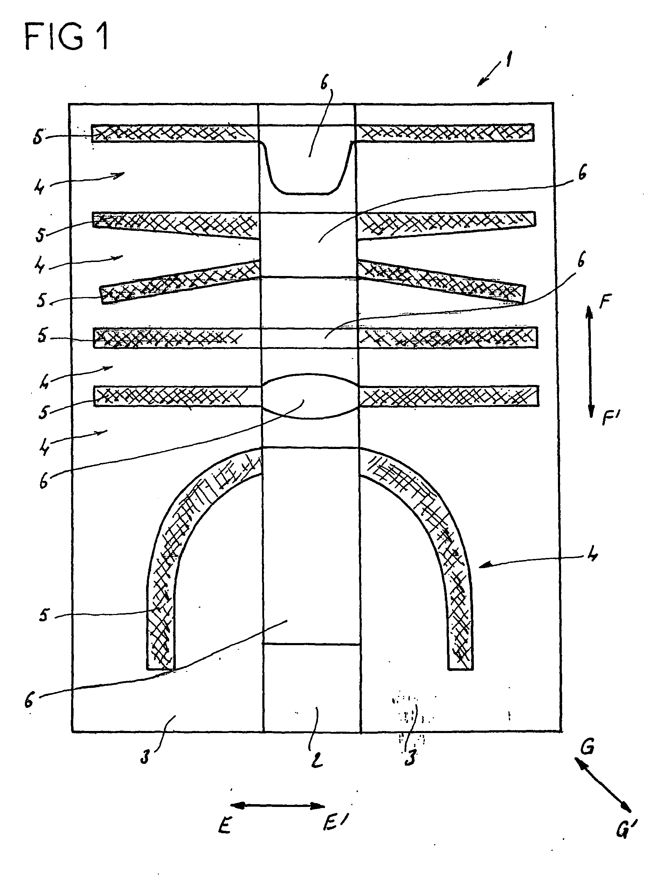

Pour chaque figure 1 à 6, on définit par E-E' la direction ou dimension transversale du tricot, par F-F' la direction ou dimension longitudinale du tricot et par G-G' la direction ou dimension diagonale du tricot.

En se référant à la figure 1, est représenté un tricot prothétique 1 selon l'invention comprenant une bande centrale 2 à deux nappes de base et deux bandes latérales 3 à quatre nappes, à savoir les deux nappes de base et deux nappes supplémentaires. Ce tricot 1 comporte des découpes définissant des implants 4 de soutien pour le traitement des prolapsus et de l'incontinence urinaire d'effort dont les parties centrales 6 sont découpées au niveau de la bande centrale 2 du tricot 1 et dont les extrémités 5 sont découpées au niveau respectivement des deux bandes latérales 3 du tricot 1. Ainsi, il est possible de découper des implants 4 présentant une partie centrale 6 bombée symétrique ou asymétrique. Il est possible de découper des implants 4 dont les extrémités 5 sont perpendiculaires, ou au contraires obliques, par rapport à la partie centrale 6. Il est également possible de découper des implants 4 présentant par exemple deux extrémités 5 de chaque côté de la partie centrale 6. Grâce à la construction du tricot prothétique explicitée ci-après, ces implants 4 présentent une grande souplesse, une grande porosité et une grande élasticité dans leur partie centrale 6 destinée à être en contact avec l'organe à soutenir, tandis qu'elles présentent une bonne résistance à la déchirure et au tuilage au niveau de leurs extrémités 5 destinées à être ancrées par exemple à la paroi abdominale, et ce même si ces extrémités présentent une largeur allant de 1 cm à 3 cm par exemple.

Le tricot prothétique peut aussi ne pas présenter de découpe particulière. Le chirurgien peut alors découper la forme particulière qu'il souhaite pour le façonnage d'un implant de soutien pour le traitement des prolapsus et de l'incontinence urinaire d'effort, la simplicité d'utilisation du tricot prothétique selon l'invention permettant entre autres la réalisation d'implants et de bandelettes de toutes formes, même les plus complexes.

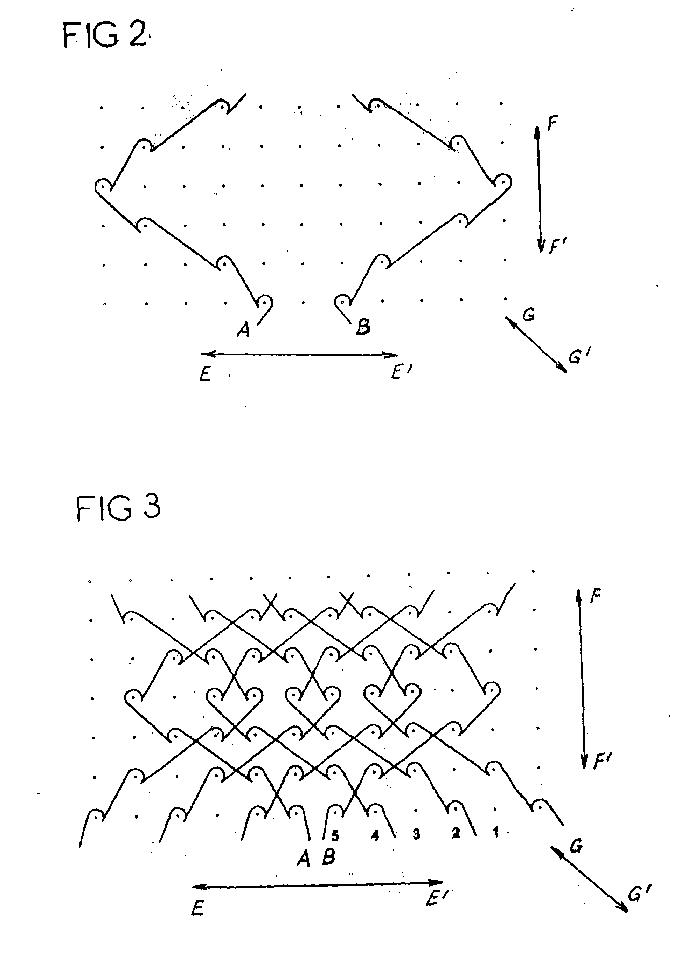

En se référant aux figures 2 et 3, sont représentés les fils avant (A) et arrière (B) formant respectivement la nappe de base avant et la nappe de base arrière réalisant le tricot de base d'un tricot prothétique selon l'invention, c'est-à-dire une première armure d'un tricot selon l'invention. Cette première armure peut s'étendre sur l'ensemble de la surface du tricot ou seulement sur la surface de la bande centrale 2 du tricot.

La figure 2 montre seulement un fil avant (A) et un fil arrière (B) pour montrer clairement le déplacement des fils. En se référant aux figures 2 et 3, le tricot représenté est réalisé sur un métier chaîne ou métier Rachel avec deux barres à passettes enfilées un plein, un vide, se déplaçant symétriquement l'une par rapport à l'autre. En référence à la figure 3, plusieurs fils avant et plusieurs fils arrière sont représentés et le motif réalisé est un atlas mailles ouvertes avec des progressions sous deux et trois aiguilles. Pour cette armure, les valeurs d'élasticité longitudinale, transversale et diagonale sont similaires.

Le barème de tricotage représenté schématiquement sur les figures 2 et 3 est le barème suivant:
- pour la nappe avant, 0-1/1-2/3-4/5-4/4-3/2-1//,
- pour la nappe arrière, 5-4/4-3/2-1/0-1/1-2/3-4//.

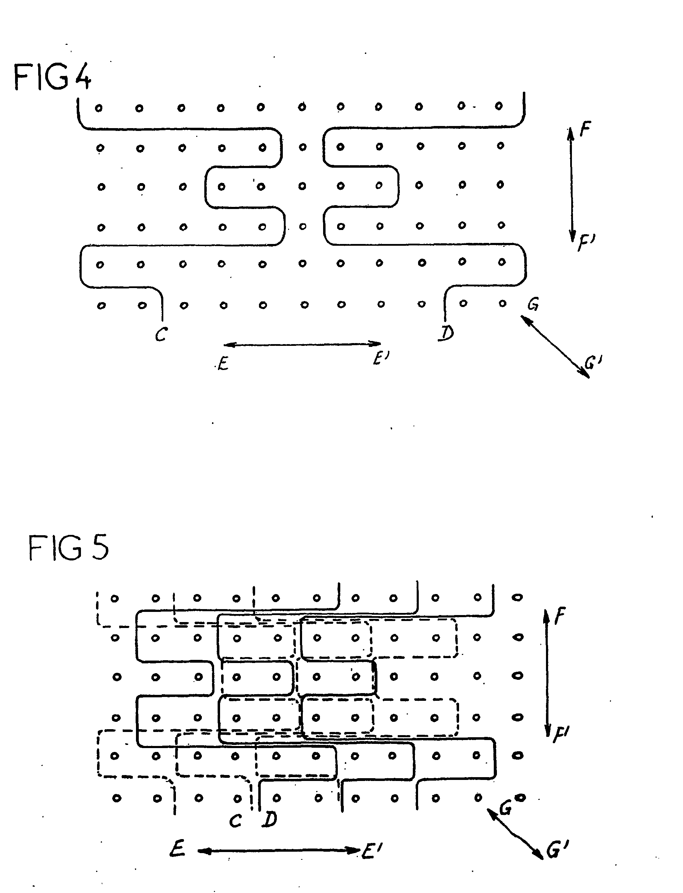

En se référant à la figure 4 sont représentés un fil (C) d'une première nappe supplémentaire et un fil (D) d'une deuxième nappe supplémentaire réalisant une deuxième armure d'un tricot prothétique selon l'invention. Cette deuxième armure s'étend seulement sur les bandes latérales 3 du tricot. En référence à la figure 5, plusieurs fils (C, D) de ces deux nappes supplémentaires sont représentés : pour une meilleure compréhension, les fils C sont représentés en traits pointillés et les fils D sont représentés en traits pleins. Il ressort clairement de cette figure pour l'homme du métier que le motif réalisé ne forme pas de mailles. Du fait de la présence de nombreux retours horizontaux des fils C et D, la résistance à la rupture dans la direction transversale de cette deuxième armure est particulièrement élevée.

Le barème de tricotage représenté schématiquement sur les figures 4 et 5 est le barème suivant:
- pour la première nappe supplémentaire, selon fil C, 3-3/5-5/0-0/2-2/0-0/5-5//,
- pour la deuxième nappe supplémentaire, selon fil D, 2-2/0-0/5-5/3-3/5-5/0-0//.

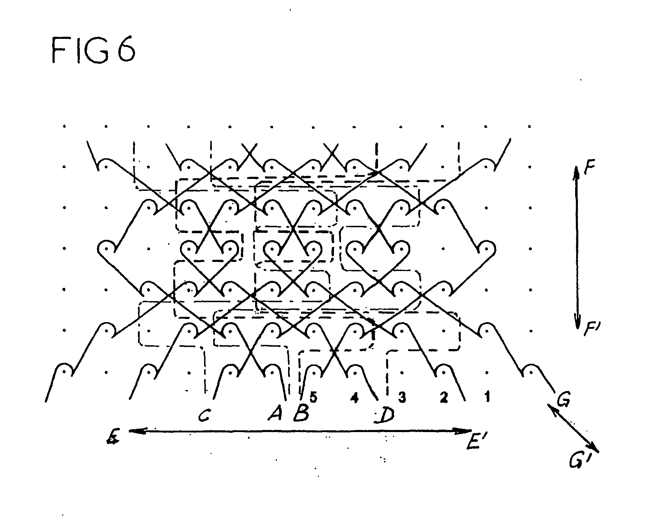

En référence à la figure 6, est représentée la combinaison de la première armure selon les figures 2 et 3 et de la deuxième armure selon les figures 4 et 5 dans le cas où la première armure s'étend sur l'ensemble de la surface du tricot. Pour une meilleure compréhension, les fils A et B sont représentés en traits pleins, les fils C sont représentés en traits mixtes et les fils D sont représentés en traits pointillés. Cette combinaison a lieu seulement à partir de la jonction entre la bande centrale et une bande latérale du tricot prothétique, jusqu'à la bordure correspondante de ce dernier. Pour cela, les deux nappes supplémentaires, définissant la deuxième armure sont réalisées sur le même métier que celui utilisé pour la première armure en rajoutant deux barres à passettes enfilées à disposition, c'est-à-dire seulement sur la largeur des bandes latérales. Ces deux barres à passettes supplémentaires se déplacent également symétriquement l'une par rapport à l'autre.

En se référant à cette figure 6, la bande centrale 2 du tricot est réalisée avec deux barres à passettes, tandis que les bandes latérales 3 du tricot sont réalisées avec quatre barres à passettes. Par ailleurs, on constate des figures 3 et 5 que tous les fils du tricot se déplacent au total sous cinq aiguilles, ce qui permet d'obtenir une meilleure tenue des fils, avec moins de risques d'effilochage des bords, et de démaillage. Enfin, on constate de la figure 6 que les trous ou alvéoles déterminés par la première armure ne sont pas bouchés par l'apport de la deuxième armure et le tricot obtenu est ainsi ajouré sur l'ensemble de ses bandes centrale et latérales.

Ainsi, au niveau des bandes latérales 3 du tricot, les fils C et D des deux nappes supplémentaires ne forment pas des mailles, mais des trames partielles, et ils sont pris à l'intérieur des mailles formées par la première armure. Cet arrangement spécifique des nappes de base et des nappes supplémentaires permet d'apporter des fils, et donc de la matière, sur les bandes latérales 3 du tricot sans créer de surépaisseur ou de rupture apparente entre la bande centrale 2 et les bandes latérales 3. Par ailleurs, du fait de cet apport de matière, de la nature particulière de la deuxième armure et de l'arrangement spécifique des nappes entre elles, le tricot présente, au niveau de chacune de ses bandes latérales 3, une résistance à la rupture et au tuilage supérieure à celle qu'il présente au niveau de sa bande centrale.

Dans le cas où la première armure ne s'étend que sur la bande centrale 2 du tricot prothétique, la bande centrale 2 est réalisée avec deux barres à passettes enfilées à disposition seulement sur la largeur de la bande centrale 2 et les bandes latérales 3 sont réalisées avec deux autres barres à passettes également enfilées à disposition mais cette fois-ci seulement sur les largeurs respectives des bandes latérales 3.

En sortie de métier, le tricot obtenu est de préférence soumis à une opération de thermofixation qui améliore encore sa stabilisation dans les directions longitudinale et transversale.

Le tricot selon l'invention autorise ainsi le découpage de fines bandelettes au niveau des bandes latérales du tricot, comme par exemple des bandelettes de moins d'1 cm sans craindre que ces bandelettes ne tuilent ou ne se déchirent sous l'effet d'une traction selon la longueur desdites bandelettes.

### EXEMPLE :

On a réalisé un tricot selon l'invention à partir d'un fil monofilament de polypropylène, de diamètre 0,10 mm, sur un métier Rachel, avec les barèmes suivants pour les différentes nappes:
- nappe de base avant : 0-1/1-2/3-4/5-4/4-3/2-1//,
- nappe de base arrière : 5-4/4-3/2-1/0-1/1-2/3-4//,
- première nappe supplémentaire : 3-3/5-5/0-0/2-2/0-0/5-5//,
- deuxième nappe supplémentaire : 2-2/0-0/5-5/3-3/5-5/0-0//.

Les barres à passettes des nappes de base avant et arrière étaient enfilées en continu, un plein, un vide. La jauge utilisée était de 24 aiguilles un plein, un vide, soit 12 aiguilles par pouce.

Les barres à passettes des deux nappes supplémentaires étaient enfilées à disposition sur les largeurs respectives des bandes latérales. A l'intérieur de chaque bande latérale, les barres à passettes étaient enfilées un plein, un vide. La jauge utilisée était de 24 aiguilles un plein, un vide, soit 12 aiguilles par pouce.

En sortie de métier, le tricot a été soumis à une opération de thermofixation.

La largeur de la bande centrale 2 était de 5 cm. La largeur de chaque bande latérale 3 était de 20 cm. On a découpé dans une bande latérale du tricot une bandelette de largeur 10 mm et de longueur 150 mm. On a ensuite mesuré la force à la rupture de cette bandelette dans la direction longitudinale du tricot, c'est-à-dire selon la largeur de la bandelette, dans la direction transversale du tricot, c'est-à-dire selon la longueur de la bandelette, et dans la direction diagonale de la bandelette. Les résultats sont les suivants :
- force à la rupture dans la direction longitudinale, mesurée selon la norme ISO 13934-1 pour laquelle la largeur de l'échantillon était de 10 mm et la longueur de l'échantillon était de 150 mm : 31 N,
- force à la rupture dans la direction transversale, mesurée selon la norme ISO 13934-1 pour laquelle la largeur de l'échantillon était de 10 mm et la longueur de l'échantillon était de 150 mm : 42 N,
- force à la rupture dans la direction diagonale, mesurée selon la norme ISO 13934-1 pour laquelle la largeur de l'échantillon était de 10 mm et la longueur de l'échantillon était de 150 mm : 33 N.

La bandelette de largeur 10 mm ainsi découpée dans une partie latérale du tricot selon l'invention présente une excellente résistance à la traction et est ainsi tout à fait adaptée à la fabrication d'un implant de soutien pour le traitement des prolapsus et de l'incontinence urinaire d'effort.

La présente invention n'est pas limitée aux formes d'exécution décrites dans la présente demande à titre d'exemples. Par exemple, la largeur de la bande centrale et celle des bandes latérales peuvent varier.

Dans une autre forme de réalisation de l'invention, la bande centrale 2 du tricot prothétique est recouverte sur au moins l'une de ses faces d'un matériau biorésorbable, par exemple par du collagène, l'implant étant ainsi particulièrement peu agressif envers l'organe à soutenir.

## Revendications

1. Tricot prothétique (1) ajouré fait d'une seule pièce, à base d'un arrangement constitué par plusieurs nappes de fils d'un matériau polymère biocompatible, comprenant, de manière continue dans le sens de la fabrication du tricot, une bande centrale (2) et deux bandes latérales (3) respectivement de part et d'autre de la bande centrale (2), l'élasticité du tricot dans la bande centrale (2) étant supérieure à l'élasticité du tricot dans chacune des bandes latérales (3), **caractérisé en ce qu'**il comprend i) au moins deux nappes de base, une nappe avant et une nappe arrière, s'étendant au moins sur l'ensemble de la surface de la bande centrale (2), lesdites deux nappes de base étant des nappes maillantes et définissant une première armure, et ii) au moins deux nappes supplémentaires, en arrière de ladite nappe de base arrière, s'étendant seulement sur les surfaces respectives des bandes latérales (3), lesdites deux nappes supplémentaires étant des nappes non maillantes de trame partielle et définissant une deuxième armure.

2. Tricot (1) selon la revendication 1, **caractérisé en ce que** les deux nappes de base s'étendent sur la totalité de la surface du tricot (1), c'est-à-dire sur la surface de la bande centrale (2) et sur les surfaces respectives des bandes latérales (3).

3. Tricot (1) selon l'une quelconque des revendications précédentes, **caractérisé en ce que** la masse surfacique du tricot (1) dans chacune des bandes latérales (3) est au moins deux fois supérieure à la masse surfacique du tricot (1) dans la bande centrale (2).

4. Tricot (1) selon l'une quelconque des revendications précédentes, **caractérisé en ce que** la force à la rupture du tricot (1) dans la direction longitudinale et dans la direction transversale au niveau de chaque bande latérale (3) est au moins deux fois supérieure à la force à la rupture du tricot dans la direction longitudinale et dans la direction transversale au niveau de la bande centrale (2).

5. Tricot (1) selon l'une quelconque des revendications précédentes, **caractérisé en ce que** la bande centrale (2) présente une masse surfacique allant de 15 à 50 g/m², et de préférence allant de 25 à 35 g/m².

6. Tricot (1) selon l'une quelconque des revendications précédentes, **caractérisé en ce que** la force à la rupture du tricot dans la bande centrale (2) est d'environ 90 N dans la direction longitudinale et dans la direction transversale.

7. Tricot (1) selon l'une quelconque des revendications précédentes, **caractérisé en ce que** chaque bande latérale (3) présente une masse surfacique allant de 60 à 80 g/m², et de préférence de l'ordre de 70 g/m².

8. Tricot (1) selon l'une quelconque des revendications précédentes, **caractérisé en ce que** la force à la rupture du tricot (1) dans chaque bande latérale (3) est d'environ 200 N dans la direction longitudinale et dans la direction transversale.

9. Tricot (1) selon l'une quelconque des revendications précédentes, **caractérisé en ce qu'**il est réalisé à base de fils, monofilaments ou multifilaments, d'un matériau polymère biocompatible choisi parmi le polypropylène, le polyester, le polyamide et leurs mélanges.

10. Tricot (1) selon l'une quelconque des revendications précédentes, **caractérisé en ce qu'**il comprend en outre d'autres nappes de fils en addition aux deux nappes de base et aux deux nappes supplémentaires.

11. Tricot selon l'une quelconque des revendications précédentes, **caractérisé en ce que** la bande centrale (2) est recouverte sur au moins l'une de ses faces d'un matériau biorésorbable.

12. Utilisation d'un tricot prothétique (1) selon l'une quelconque des revendications 1 à 11 pour obtenir un produit prothétique à usage chirurgical, notamment pour obtenir un implant de soutien comprenant une bande centrale et deux bandes latérales, (4) pour le traitement des prolapsus et de l'incontinence urinaire d'effort.

13. Implant de soutien (4) pour le traitement des prolapsus et de l'incontinence urinaire d'effort comprenant une bande centrale et deux bandes latérales, obtenu par découpage d'un tricot prothétique (1) selon l'une quelconque des revendications 1 à 11.

14. Implant (4) selon la revendication 13, **caractérisé en ce qu'**il présente une partie centrale (6) bombée symétrique ou asymétrique.

15. Implant (4) selon la revendication 13 ou 14, **caractérisé en ce que** ses extrémités (5) sont perpendiculaires par rapport à la partie centrale (6).

16. Implant (4) selon la revendication 13 ou 14, **caractérisé en ce que** ses extrémités (5) sont obliques par rapport à la partie centrale (6).

17. Implant (4) selon l'une quelconque des revendications 13 à 16, **caractérisé en ce qu'**il présente deux extrémités (5) de chaque côté de la parties centrale (6).

18. Procédé de fabrication d'un tricot prothétique (1) ajouré fait d'une seule pièce, à base d'un arrangement constitué par plusieurs nappes de fils d'un matériau polymère biocompatible, comprenant, de manière continue, dans le sens de la fabrication du tricot, une bande centrale (2) et deux bandes latérales (3), respectivement de part et d'autre de la bande centrale, l'élasticité du tricot dans la bande centrale (2) étant supérieure à l'élasticité du tricot dans chacune des bandes latérales (3), **caractérisé en ce qu'**il comprend l'étape suivante :
- fabrication d'un tricot (1) sur un métier chaîne ou Rachel selon deux nappes de base, une nappe de base avant et une nappe de base arrière définissant une première armure, et deux nappes supplémentaires définissant une deuxième armure, les deux nappes de base étant enfilées en continu ou à disposition au moins sur la largeur de la bande centrale (1), chacune des nappes de base étant obtenue à partir d'une barre à passettes, le barème suivi pour le tricotage des fils de chaque nappe de base conduisant à la formation de mailles, les deux nappes supplémentaires étant enfilées à disposition seulement sur les largeurs respectives des bandes latérales (3), chacune des nappes supplémentaires étant obtenue à partir d'une barre à passettes, le barème suivi pour le tricotage des fils de chaque nappe supplémentaire conduisant à une trame partielle non maillante.

19. Procédé selon la revendication 18, **caractérisé en ce que** le barème suivi pour le tricotage des fils de chaque nappe de base conduit à la formation d'un motif atlas à mailles ouvertes.

20. Procédé selon la revendication 19, **caractérisé en ce que** ledit barème conduit à la formation d'un motif atlas à mailles ouvertes irrégulier.

21. Procédé selon la revendication 20, **caractérisé en ce que** les fils de la nappe de base avant sont tricotés selon un barème 0-1/1-2/3-4/5-4/4-3/2-1// et les fils de la nappe de base arrière sont tricotés selon un barème 5-4/4-3/2-1/0-1/1-2/3-4//.

22. Procédé selon l'une quelconque des revendications 18 à 21, **caractérisé en ce que** les deux barres à passette des nappes de base sont enfilées en continu un plein, un vide, et se déplacent symétriquement l'une par rapport à l'autre.

23. Procédé selon la revendication 22, **caractérisé en ce que** les deux barres à passettes des nappes supplémentaires se déplacent en trame partielle, avec des jetées « sous » en rapport avec la première armure.

24. Procédé selon la revendication 23, **caractérisé en ce que** les deux barres à passette des nappes supplémentaires se déplacent en trame partielle avec des jetées « sous » deux et cinq aiguilles.

25. Procédé selon la revendication 24, **caractérisé en ce que** les fils de l'une des deux nappes supplémentaires sont tricotés selon le barème 3-3/5-5/0-0/2-2/0-0/5-5//, et les fils de l'autre nappe supplémentaire sont tricotés selon le barème 2-2/0-0/5-5/3-3/5-5/0-0//*.*

26. Procédé selon l'une quelconque des revendications 23 à 25, **caractérisé en ce que,** à l'intérieur de chaque bande latérale, les deux barres à passettes des nappes supplémentaires sont enfilées un plein, un vide.

27. Procédé selon l'une quelconque des revendications 18 à 26, **caractérisé en ce qu'**on rajoute sur le métier des barres à passettes additionnelles pour obtenir autant de nappes de fils additionnelles.

28. Procédé selon l'une quelconque des revendications 18 à 27, **caractérisé en ce que**, en sortie de métier, le tricot est soumis à une opération de thermofixation.

## Claims

1. Openwork prosthetic knit (1) made in one piece, based on an arrangement formed by several sheets of yarns of a biocompatible polymer material, comprising, in a continuous manner in the direction of production of the knit, a central band (2) and two lateral bands (3) on either side, respectively, of the central band (2), the elasticity of the knit in the central band (2) being greater than the elasticity of the knit in each of the lateral bands (3), **characterized in that** it comprises i) at least two base sheets, a front sheet and a rear sheet, extending at least across the whole surface of the central band (2), said two base sheets being meshing sheets and defining a first weave, and ii) at least two supplementary sheets, behind said rear base sheet, extending only across the respective surfaces of the lateral bands (3), said two supplementary sheets being non-meshing sheets of partial weft and defining a second weave.

2. Knit (1) according to Claim 1, **characterized in that** the two base sheets extend across the whole surface of the knit (1), that is to say across the surface of the central band (2) and across the respective surfaces of the lateral bands (3).

3. Knit (1) according to either one of the preceding claims, **characterized in that** the mass per unit area of the knit (1) in each of the lateral bands (3) is at least twice as great as the mass per unit area of the knit (1) in the central band (2).

4. Knit (1) according to any one of the preceding claims, **characterized in that** the breaking strength of the knit (1) in the longitudinal direction and in the transverse direction in the area of each lateral band (3) is at least twice as great as the breaking strength of the knit in the longitudinal direction and in the transverse direction in the area of the central band (2).

5. Knit (1) according to any one of the preceding claims, **characterized in that** the central band (2) has a mass per unit area ranging from 15 to 50 g/m², and preferably ranging from 25 to 35 g/m².

6. Knit (1) according to any one of the preceding claims, **characterized in that** the breaking strength of the knit in the central band (2) is about 90 N in the longitudinal direction and in the transverse direction.

7. Knit (1) according to any one of the preceding claims, **characterized in that** each lateral band (3) has a mass per unit area ranging from 60 to 80 g/m², and preferably of the order of 70 g/m².

8. Knit (1) according to any one of the preceding claims, **characterized in that** the breaking strength of the knit (1) in each lateral band (3) is about 200 N in the longitudinal direction and in the transverse direction.

9. Knit (1) according to any one of the preceding claims, **characterized in that** it is formed from yarns, monofilaments or multifilaments, of a biocompatible polymer material chosen from among polypropylene, polyester, polyamide and mixtures thereof.

10. Knit (1) according to any one of the preceding claims, **characterized in that** it additionally comprises other sheets of yarns in addition to the two base sheets and to the two supplementary sheets.

11. Knit according to any one of the preceding claims, **characterized in that** the central band (2) is covered, on at least one of its faces, with a bioabsorbable material.

12. Use of a prosthetic knit (1) according to any one of Claims 1 to 11 for obtaining a prosthetic product for surgical use, in particular for obtaining a support implant (4) comprising a central band and two lateral bands for treating prolapses and stress urinary incontinence.

13. Support implant (4) for treating prolapses and stress urinary incontinence, comprising a central band and two lateral bands, obtained by cutting a prosthetic knit (1) according to any one of Claims 1 to 11.

14. Implant (4) according to Claim 13, **characterized in that** it has a symmetrical or asymmetrical bulged central part (6).

15. Implant (4) according to Claim 13 or 14, **characterized in that** its ends (5) are perpendicular with respect to the central part (6).

16. Implant (4) according to Claim 13 or 14, **characterized in that** its ends (5) are oblique with respect to the central part (6).

17. Implant (4) according to any one of Claims 13 to 16, **characterized in that** it has two ends (5) on each side of the central part (6).

18. Method for producing an openwork prosthetic knit (1) made in one piece, based on an arrangement formed by several sheets of yarns of a biocompatible polymer material, comprising, in a continuous manner in the direction of production of the knit, a central band (2) and two lateral bands (3) on either side, respectively, of the central band, the elasticity of the knit in the central band (2) being greater than the elasticity of the knit in each of the lateral bands (3), **characterized in that** it comprises the following step:
- production of a knit (1) on a warp-knitting machine or Rachel loom, with two base sheets, a front base sheet and a rear base sheet defining a first weave, and two supplementary sheets defining a second weave, the two base sheets being threaded continuously or intermittently at least across the width of the central band (1), each of the base sheets being obtained from one guide bar, the chart followed for knitting the yarns of each base sheet leading to the formation of meshes, the two supplementary sheets being threaded intermittently only across the respective widths of the lateral bands (3), each of the supplementary sheets being obtained from one guide bar, the chart followed for knitting the yarns of each supplementary sheet leading to a non-meshing partial weft.

19. Method according to Claim 18, **characterized in that** the chart followed for knitting the yarns of each base sheet leads to the formation of an atlas pattern with open meshes.

20. Method according to Claim 19, **characterized in that** said chart leads to the formation of an irregular open-mesh atlas pattern.

21. Method according to Claim 20, **characterized in that** the yarns of the front base sheet are knitted according to a chart 0-1/1-2/3-4/5-4/4-3/2-1// and the yarns of the rear base sheet are knitted according to a chart 5-4/4-3/2-1/0-1/1-2/3-4//.

22. Method according to any one of Claims 18 to 21, **characterized in that** the two guide bars of the base sheets are threaded continuously, one full, one empty, and move symmetrically with respect to one another.

23. Method according to Claim 22, **characterized in that** the two guide bars of the supplementary sheets move in partial weft, with throws "underneath" in relation to the first weave.

24. Method according to Claim 23, **characterized in that** the two guide bars of the supplementary sheets move in partial weft with throws "underneath" two and five needles.

25. Method according to Claim 24, **characterized in that** the yarns of one of the two supplementary sheets are knitted according to the chart 3-3/5-5/0-0/2-2/0-0/5-5//, and the yarns of the other supplementary sheet are knitted according to the chart 2-2/0-0/5-5/3-3/5-5/0-0//.

26. Method according to any one of Claims 23 to 25, **characterized in that,** inside each lateral band, the two guide bars of the supplementary sheets are threaded one full, one empty.

27. Method according to any one of Claims 18 to 26, **characterized in that** additional guide bars are added to the loom in order to obtain as many additional sheets of yarns.

28. Method according to any one of Claims 18 to 27, **characterized in that,** on leaving the loom, the knit is subjected to a thermosetting operation.

## Patentansprüche

1. Prothetisches durchbrochenes Gewirk (1), das auf der Basis einer Anordnung, die durch mehrere Fadenlagen eines biokompatiblen polymeren Materials gebildet ist, einstückig gefertigt ist, und das, im Sinn der Herstellung des Gewirks in zusammenhängender Weise, einen mittigen Streifen (2) und zwei seitliche Streifen (3) beidseits des mittigen Streifens (2) aufweist, wobei die Elastizität des Gewirkes im mittigen Streifen (2) größer ist als die Elastizität des Gewirks in jedem der seitlichen Streifen (3), **dadurch gekennzeichnet, dass** es aufweist: i) zumindest zwei Basislagen, und zwar eine vordere Lage und eine hintere Lage, die sich zumindest über die Gesamtheit der Oberfläche des mittigen Streifens (2) erstrecken, wobei die zwei Basislagen maschige Lagen sind und eine erste Bindung definieren, und ii) zumindest zwei ergänzende Lagen hinter der hinteren Basislage, die sich nur auf den jeweiligen Oberflächen der seitlichen Streifen (3) erstrecken, wobei die zwei ergänzenden Lagen nicht-maschige Lagen von teilweisem Schuss sind und eine zweite Bindung definieren.

2. Gewirk (1) nach Anspruch 1, **dadurch gekennzeichnet, dass** die zwei Basislagen sich über die Gesamtheit der Oberfläche des Gewirkes (1) erstrecken, das heißt über die Oberfläche des mittigen Streifens (2) und über die jeweiligen Oberflächen der seitlichen Streifen (3).

3. Gewirk (1) nach einem der vorhergehenden Ansprüche, **dadurch gekennzeichnet, dass** die flächenbezogene Masse des Gewirks (1) in jedem der seitlichen Streifen (3) zumindest zweimal größer ist als die flächenbezogene Masse des Gewirks (1) in dem mittigen Streifen (2).

4. Gewirk (1) nach einem der vorhergehenden Ansprüche, **dadurch gekennzeichnet, dass** die Reißkraft des Gewirks (1) in der Längsrichtung und in der Querrichtung im Bereich jedes seitlichen Streifens (3) zumindest zweimal größer ist als die Reißkraft des Gewirks in der Längsrichtung und in der Querrichtung im Bereich des mittigen Streifens (2).

5. Gewirk (1) nach einem der vorhergehenden Ansprüche, **dadurch gekennzeichnet, dass** der mittige Streifen (2) eine flächenbezogene Masse aufweist, die von 15 bis 50 g/m², und vorzugsweise von 25 bis 35 g/m² reicht.

6. Gewirk (1) nach einem der vorhergehenden Ansprüche, **dadurch gekennzeichnet, dass** die Reißkraft des Gewirks in dem mittigen Streifen (2) etwa 90 N in der Längsrichtung und in der Querrichtung beträgt.

7. Gewirk (1) nach einem der vorhergehenden Ansprüche, **dadurch gekennzeichnet, dass** jeder seitliche Streifen (3) eine flächenbezogene Masse aufweist, die von 60 bis 80 g/m² reicht, und vorzugsweise in der Größenordnung von 70 g/m² beträgt.

8. Gewirk (1) nach einem der vorhergehenden Ansprüche, **dadurch gekennzeichnet, dass** die Reißkraft des Gewirks (1) in jedem seitlichen Streifen (3) etwa 200 N in der Längsrichtung und in der Querrichtung beträgt.

9. Gewirk (1) nach einem der vorhergehenden Ansprüche, **dadurch gekennzeichnet, dass** es auf der Basis von Fäden, Monofilamenten oder Multifilamenten aus einem biokompatiblen polymeren Material hergestellt ist, das aus Polypropylen, Polyester, Polyamid und ihren Gemischen ausgewählt ist.

10. Gewirk (1) nach einem der vorhergehenden Ansprüche, **dadurch gekennzeichnet, dass** es außerdem weitere Fadenlagen zusätzlich zu den zwei Basislagen und zu den zwei ergänzenden Lagen aufweist.

11. Gewirk nach einem der vorhergehenden Ansprüche, **dadurch gekennzeichnet, dass** der mittige Streifen (2) zumindest auf einer seiner Seiten mit einem bioresorbierbaren Material bedeckt ist.

12. Verwendung eines prothetischen Gewirks (1) nach einem der Ansprüche 1 bis 11, um ein prothetisches Erzeugnis für den chirurgischen Gebrauch zu erhalten, insbesondere um ein Stützimplantat (4) für die Behandlung von Prolapsen und Stressharninkontinenz zu erhalten, das einen mittigen Streifen und zwei seitliche Streifen aufweist.

13. Stützimplantat (4) für die Behandlung von Prolapsen und Stresshärninkontinenz, das einen mittigen Streifen und zwei seitliche Streifen aufweist, das durch Zuschneiden eines prothetischen Gewirks (1) gemäß einem der Ansprüche 1 bis 11 erhalten worden ist.

14. Implantat (4) nach Anspruch 13, **dadurch gekennzeichnet, dass** es einen symmetrischen oder asymmetrischen gewölbten mittigen Teil (6) aufweist.

15. Implantat (4) nach Anspruch 13 oder 14, **dadurch gekennzeichnet, dass** seine Enden (5) rechtwinklig bezüglich des mittigen Teils (6) sind.

16. Implantat (4) nach Anspruch 13 oder 14, **dadurch gekennzeichnet, dass** seine Enden (5) schräg bezüglich des mittigen Teils (6) sind.

17. Implantat (4) nach einem der Ansprüche 13 bis 16, **dadurch gekennzeichnet, dass** es zwei Enden (5) auf jeder Seite des mittigen Teils (6) aufweist.

18. Verfahren zum Herstellen eines durchbrochenen prothetischen Gewirks (1), das auf der Basis einer Anordnung, die durch mehrere Fadenlagen aus einem biokompatiblen polymeren Material gebildet ist, einstückig gefertigt ist, und das, im Sinn der Herstellung des Gewirks in zusammenhängender Weise, einen mittigen Streifen (2) und zwei seitliche Streifen (3) beidseits des mittigen Streifens aufweist, wobei die Elastizität des Gewirks in dem mittigen Streifen (2) größer ist als die Elastizität des Gewirks in jedem der seitlichen Streifen (3), **dadurch gekennzeichnet, dass** es den folgenden Schritt aufweist:
- Herstellen eines Gewirks (1) auf einer Kettenwirk- oder Rachelmaschine gemäß zweier Basislagen, und zwar einer vorderen Basislage und einer hinteren Basislage, die eine erste Bindung definieren, und zweier ergänzender Lagen, die eine zweite Bindung definieren, wobei die beiden Basislagen durchgehend oder zur Verfügung stehend zumindest auf der Breite des mittigen Streifens (1) eingefädelt werden, wobei jede der Basislagen ausgehend von einem Legebarren erhalten wird, wobei das für das Wirken der Fäden jeder Basislage fortlaufende Schema zur Bildung von Maschen führt, wobei die zwei ergänzenden Lagen zur Verfügung stehend nur auf den jeweiligen Breiten der seitlichen Streifen (3) eingefädelt werden, wobei jede der ergänzenden Lagen ausgehend von einem Legebarren erhalten wird, wobei das für das Wirken der Fäden jeder ergänzenden Lage fortlaufende Schema zu einem nicht-maschigen teilweisen Schuss führt.

19. Verfahren nach Anspruch 18, **dadurch gekennzeichnet, dass** das zum Wirken der Fäden jeder Basislage fortlaufende Schema zur Bildung eines Atlasmotivs mit offenen Maschen führt.

20. Verfahren nach Anspruch 19, **dadurch gekennzeichnet, dass** das Schema zur Bildung eines Atlasmotivs mit unregelmäßigen offenen Maschen führt.

21. Verfahren nach Anspruch 20, **dadurch gekennzeichnet, dass** die Fäden der vorderen Basislage gemäß einem Schema 0-1/1-2/3-4/5-4/4-3/2-1// gewirkt sind, und die Fäden der hinteren Basislage gemäß einem Schema 5-4/4-3/2-1/0-1/1-2/3-4// gewirkt sind.

22. Verfahren nach einem der Ansprüche 18 bis 21, **dadurch gekennzeichnet, dass** die Legebarren der Basislagen durchgehend 'einer voll, einer leer' eingefädelt sind und sich symmetrisch zueinander verschieben.

23. Verfahren nach Anspruch 22, **dadurch gekennzeichnet, dass** die zwei Legebarren der ergänzenden Lagen sich im teilweisen Schuss mit "Unterlegungen" entsprechend der ersten Bindung bewegen.

24. Verfahren nach Anspruch 23, **dadurch gekennzeichnet, dass** die zwei Legebarren der ergänzenden Lagen sich im teilweisen Schuss mit "Unterlegungen" von zwei und fünf Nadeln bewegen.

25. Verfahren nach Anspruch 24, **dadurch gekennzeichnet, dass** die Fäden der einen der zwei ergänzenden Lagen gemäß dem Schema 3-3/5-5/0-0/2-2/0-0/5-5// gewirkt sind, und die Fäden der anderen ergänzenden Lage gemäß dem Schema 2-2/0-0/5-5/3-3/5-5/0-0// gewirkt sind.

26. Verfahren nach einem der Ansprüche 23 bis 25, **dadurch gekennzeichnet, dass** im Inneren jedes seitlichen Streifens die zwei Legebarren der ergänzenden Lagen einer voll, einer leer' eingefädelt sind.

27. Verfahren nach einem der Ansprüche 18 bis 26, **dadurch gekennzeichnet, dass** auf der Wirkmaschine zusätzliche Legebarren hinzugefügt werden, um ebenso viele zusätzliche Fadenlagen zu erhalten.

28. Verfahren nach einem der Ansprüche 18 bis 27, **dadurch gekennzeichnet, dass** am Ausgang der Wirkmaschine das Gewirk einem Thermofixiervorgang unterzogen wird.
